(19) Europäisches Patentamt
European Patent Office
Office européen des brevets

(11) Veröffentlichungsnummer : **0 382 076 B1**

(12) # EUROPÄISCHE PATENTSCHRIFT

(45) Veröffentlichungstag der Patentschrift : **20.01.93 Patentblatt 93/03**

(21) Anmeldenummer : **90101946.3**

(22) Anmeldetag : **01.02.90**

(51) Int. Cl.$^5$ : **C07C 251/86,** C07D 215/227, C07C 255/61, C07D 317/32, C07C 255/58, C07C 259/10, C07F 9/11, C07C 309/45, C07D 295/18, A61K 31/15, A61K 31/33

(54) **Phenylhydrazone, ihre Herstellung und daraus hergestellte Arzneimittel und Kosmetika.**

Die Akte enthält technische Angaben, die nach dem Eingang der Anmeldung eingereicht wurden und die nicht in dieser Patentschrift enthalten sind.

(30) Priorität : **10.02.89 DE 3903990**

(43) Veröffentlichungstag der Anmeldung : **16.08.90 Patentblatt 90/33**

(45) Bekanntmachung des Hinweises auf die Patenterteilung : **20.01.93 Patentblatt 93/03**

(84) Benannte Vertragsstaaten : **AT BE CH DE DK ES FR GB GR IT LI NL SE**

(56) Entgegenhaltungen :
**CH-A- 498 090**
**US-A- 3 809 675**
**CHEMICAL ABSTRACTS, Band 105, Nr. 5, 4. August 1986, Seite 673, Zusammenfassung Nr. 42099f, Columbus, Ohio, US; W. JUGELT etal: "Electrochemical synthesis of N-heterocycles. II. Investigations of anodic oxidation of benylidenephenylhydrazines"**

(56) Entgegenhaltungen :
**CHEMICAL ABSTRACTS, Band 89, Nr. 21, 20. November 1987, Seite 531, Zusammenfassung Nr. 179065n, Columbus, Ohio, US; H. G.AURICH et al: "Aminyl oxide (nitroxide). XXVI. ESR spectroscopic examination of hydrazonyl oxides. Determination of the spindensity distribution"**
**CHEMICAL ABSTRACTS, Band 71, Nr, 23, 8. Dezember 1969, Seiten 331,332, Zusammenfassung Nr. 112535a, Columbus, Ohio, US; A. A.VOLOD'KIN et al: "Effect of substituent nature in bromoquinolide compounds on reaction with Grignard reagents"**

(73) Patentinhaber : **BASF Aktiengesellschaft**
**Carl-Bosch-Strasse 38**
**W-6700 Ludwigshafen (DE)**

(72) Erfinder : **Janssen, Bernd, Dr.**
**Leuschnerstrasse 18 a**
**W-6700 Ludwigshafen (DE)**
Erfinder : **Wuest, Hans-Heiner, Dr.**
**Unteres Bieth 10**
**W-6915 Dossenheim (DE)**
Erfinder : **Murray, William V.**
**Township Line Road**
**Belle Mead, New Jersey 08502 (US)**
Erfinder : **Wachter, Michael P.**
**52 North Street**
**Bloomsbury, New Jersey 08804 (US)**
Erfinder : **Bell, Stanley**
**732 Braeburn Lane**
**Narbeth, Pennsylvania 19072 (US)**

EP 0 382 076 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß Stilbenderivate [vgl. US 4 326 055, GB 21 64 938 und US 4 588 750], bei welchen die Polyenstruktur der Substanzen des Vitamin-A-Typs in aromatischen Ringen fixiert vorliegt, pharmakologische Wirkungen bei der topischen und systemischen Therapie von Neoplasien, Akne, Psoriasis und anderen dermatologischen Affektionen aufweisen. Die Wirkung dieser Verbindungen befriedigt jedoch nicht immer [vgl. G.L. Peck in: The Retinoids, Vol. II, 391-409, Ed.: M.B. Sporn et al., Academic Press N.Y. (1984), oder R.Marks et al., Med. J. Australia 146, 374-377 (1987), oder C.E. Orfanos et al., Drugs 34, 459-503 (1987)].

Der Erfindung lag die Aufgabe zugrunde, Verbindungen mit besserem Wirkspektrum zu entwickeln.

Überraschend wurde nun gefunden, daß die neuen Phenylhydrazone der Formel I

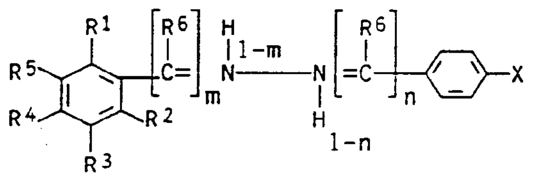

I,

in der

$R^1$, $R^2$ und $R^3$ unabhängig voneinander ein Wasserstoff-oder Halogenatom, eine $C_1$-$C_4$-Alkylgruppe, eine Hydroxyl-oder $C_1$-$C_4$-Alkoxygruppe oder den Acetoxyrest,

$R^4$ ein Wasserstoffatom, eine Hydroxylgruppe oder einen tert.-Butyl- oder $C_1$-$C_6$-Alkoxy- oder $C_2$-$C_6$-Alkoxyalkylrest,

$R^5$ ein Wasserstoffatom oder eine $C_1$-$C_4$-Alkylgruppe, oder

$R^4$ und $R^5$ unter Bildung eines Zyklus gemeinsam eine $-C(CH_3)_2$-A-$C(CH_3)_2$-Gruppe (mit A in der Bedeutung von $-CH_2CH_2-$, $-CH(CH_3)-$, $-CH_2C(O)-$ oder $-CH_2CHOH-$) oder eine $-(CH_2)_3C(CH_3)_2-$, eine $-OCH_2CH_2C(CH_3)_2-$, eine $-C(CH_3)_2CH(CH_3)CH_2C(CH_3)_2-$oder eine $-NHC(O)CH_2C(CH_3)_2$-Gruppe, wobei, wenn $R^1$, $R^2$ und $R^3$ Wasserstoff sind, $R^4$ und $R^5$ gemeinsam einen Zyklus der genannten Art bilden oder $R^4$ einen verzweigten $C_4$-$C_6$-Alkoxy- oder Alkoxyalkylrest darstellt,

$R^6$ ein Wasserstoffatom, den Methyl-, Ethyl- oder Cyclopropylrest,

m und n verschieden voneinander sind und 0 oder 1,

X ein Wasserstoffatom, eine Nitro-, Methoxy- oder Nitrilgruppe, den Sulfonsäurerest oder die Reste $-CONR^7OR^7$, $-CO_2R^7$, $-PO(OR^8)_2$, $S(O)_n$ $R^8$ (mit n = 0, 1 oder 2), $-SO_2-NR^9R^{10}$ oder $-CONR^9R^{10}$, worin $R^7$ in der Bedeutung von Wasserstoff, einer $C_1$-$C_3$-Alkyl- oder einer Phenylgruppe, die gegebenenfalls durch ein bis zwei Amino-$C_1$-$C_4$-Acylamino-, -Alkyl- oder Alkoxygruppen substituiert ist, und $R^8$ in der Bedeutung einer $C_1$-$C_3$-Alkylgruppe steht, $R^9$ und $R^{10}$ unabhängig voneinander Wasserstoff oder eine $C_1$-$C_4$-Alkylgruppe bedeuten oder gemeinsam einen Piperidin-, Piperazin-, Morpholin- oder Thiomorpholinring bilden, bedeuten, wobei, wenn X ein Wasserstoffatom, eine Methoxy-, Nitril- oder eine Nitrogruppe oder wenn X = Carboxyl und $R^1$ oder $R^2$ = Hydroxyl ist, $R^4$ und $R^5$ gemeinsam einen Zyklus der obengenannten Art bilden oder $R^3$ und $R^5$ jeweils i-Propyl oder iso- oder tert-Butyl darstellen, und wenn m=0 ist, $R^4$ keine Hydroxylgruppe darstellt,

sowie deren physiologisch verträgliche Salze ein besseres Wirkspektrum besitzen.

Von den Verbindungen der Formel I sind diejenigen bevorzugt, in denen - falls $R^1$ und $R^2$ Wasserstoff bedeuten - $R^3$ und $R^5$ für einen vorzugsweise verzweigten Alkylrest stehen oder $R^4$ und $R^5$ gemeinsam einen Zyklus ausbilden.

Bedeuten $R^1$, $R^2$ oder $R^3$ Halogen, so sind Fluor und Chlor bevorzugt.

Bevorzugt sind ferner Verbindungen der Formel I, in denen $R^6$ für ein Wasserstoffatom oder eine Methylgruppe steht, sowie Verbindungen der Formel I, worin X die Bedeutung einer $-CO_2R^7-$, einer $-CONR^7OR^7-$, $-S(O)_nR^8-$, $SO_2NR^9R^{10}-$ oder $CONR^9R^{10}$-Gruppe zukommt, wobei $R^7$ in der Bedeutung von Wasserstoff und $R^8$ in der Bedeutung einer Methyl- oder Ethylgruppe ganz besonders bevorzugt sind.

Die neuen Verbindungen der Formel I enthalten teilweise chirale Zentren und fallen im allgemeinen als Diastereomerengemische, bzw. Racemate an. Die so erhaltenen Diastereomeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) werden von der vorliegenden Erfindung umfaßt. Als therapeutische oder kosmetische Mittel kann man sowohl die einheitlichen Diastereomeren bzw. Enantiomeren wie auch deren Gemische verwenden.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden.

Geeignete Salze sind beispielsweise Ammonium-, Alkalimetall-, insbesondere des Natriums, Kaliums und Lithiums, oder Erdalkalimetallsalze, insbesondere des Calciums oder Magnesiums, sowie Salze mit geeigneten organischen Basen, wie mit niederen Alkylaminen, z.B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan sowie mit Piperidin oder Morpholin.

Gegenstand der vorliegenden Erfindung ist ferner ein Verfahren zur Herstellung der oben angegebenen Verbindungen der Formel I, indem man

a) Carbonylverbindungen der Formel IIa

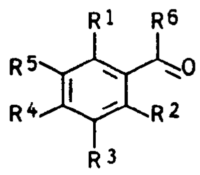

IIa,

in der $R^1$-$R^6$ die oben angegebene Bedeutung haben, mit Phenylhydrazinen der Formel IIIa

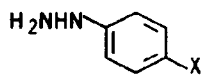

IIIa,

in der X die oben angegebene Bedeutung zukommt, oder

b) Carbonylverbindungen der Formel IIb

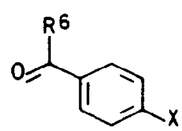

IIb,

in der $R^6$ und X die oben angegebenen Bedeutungen haben, mit Phenylhydrazinen der Formel IIIb

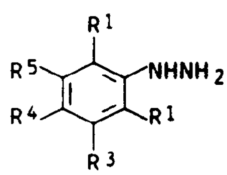

IIIb,

in der $R^1$-$R^5$ die oben angegebenen Bedeutungen haben, kondensiert.

Die Reaktion erfolgt in an sich bekannter Weise [vgl. z.B. "Methoden der Organischen Chemie" Hrsg. Eugen Müller, Bd. VII, 1, S. 461-466, Thieme Verlag, Stuttgart 1954 und Bd. VII, 2b, S, 1954-1957, Thieme Verlag, Stuttgart 1976 und Bd. X, 2, S. 410 - 414, Thieme Verlag, Stuttgart 1967] gegebenenfalls in Gegenwart eines Lösungs- oder Verdünnungsmittels, gegebenenfalls unter Zusatz eines Katalysators und gegebenenfalls unter Verwendung eines wasserbindenden Mittels bei Temperaturen zwischen 10°C und dem Siedepunkt des Gemisches, wobei man bevorzugt äquimolare Mengen der Reaktanten II und III oder eine Komponente gegebenenfalls im Überschuß von bis zu 15 Mol% umsetzt.

Zu den bevorzugten Lösungs- und Verdünnungsmitteln gehören Kohlenwasserstoffe, wie Heptan, Cyclohexan, Toluol oder Xylol, ferner niedere aliphatische Alkohole, wie Methanol, Ethanol und Isopropanol, aber auch Cyclohexanol sowie Ethylenglykol, seine Mono- und Dialkylether, Glycerin, ferner Ether wie Diethylether, Diisopropylether und Methyl-tert.-butylether oder Tetrahydrofuran und Dioxan. Des weiteren zu nennen sind Essigsäure, Amide wie Dimethylformamid oder N-Methylpyrrolidon, ferner Pyridin, Sulfolan und Wasser oder entsprechende Gemische.

Als Reaktionsbeschleuniger kommen in Frage Mineralsäuren, wie Salzsäure oder Schwefelsäure, vorzugsweise Carbonsäuren wie Essigsäure oder Trifluoressigsäure sowie deren Alkalisalze. Aber auch Basen wie Pyridin oder Morpholin können als Katalysator dienen.

Als wasserbindende Mittel sind anorganische Salze wie wasserfreies Natriumcarbonat oder Magnesiumsulfat oder auch Molekularsiebe zu verwenden; gegebenenfalls wird beim Arbeiten in liphophilen Medien das gebildete Reaktionswasser ausgekreist.

Die Reaktion wird drucklos oder unter Druck durchgeführt.

Die Ausgangsverbindungen der Formel II sind teilweise bekannt (z.B. DE-OS 3 602 473, DE-OS 3 434 942, DE-OS 3 434 944) oder werden erhalten nach üblichen Methoden zur Darstellung von Arylalkylketonen, beispielsweise durch Friedel-Crafts Acylierung (H.O. House: "Modern Synthetic Reactions", 2nd Ed., W.A. Benjamin Inc. Menlo Park, CA; 1972, dort S. 797 ff und dort zitierte Literatur) oder durch Oxidation der entsprechenden Alkylbenzole (H.O. House, l.c., S. 288 f und dort angegebene Literatur) sowie zur Darstellung von Benzaldehyden, beispielsweise durch Aromatenformylierung nach Vilsmeier (vgl. De. Meheas, Bull. Soc. Chem. Fr. 1989-1999 (1962) und dort zitierte Literatur) oder durch Reduktion der entsprechenden Benzoylhalogenide (vgl. Fuson in: Patai, "The Chemistry of the Carbonyl Group", Vol. 1, S. 211-232, Interscience Publ., N.Y. 1966 oder Wheeler in Patai, "The Chemistry of Acyl Halides", S. 231-251, Interscience Publ. N.Y. 1972) oder Benzonitrile (vgl. J. March: "Advanced Organic Chemistry, 2nd Ed., McGraw-Hill Kogakusha LTD. Tokyo, 1977, S. 835-836 und dort zitierte Literatur).

Die Ausgangsverbindungen der Formel III sind bekannt (vgl. Methoden der Organischen Chemie" Hrsg. Eugen Müller, Bd. X, 2, S. 169-315, Thieme Verlag, Stuttgart 1967) oder lassen sich herstellen nach allgemein bekannten Verfahren zur Darstellung von Arylhydrazinen.

Die erfindungsgemäßen Verbindungen und ihre physiologisch verträglichen Salze können aufgrund ihrer pharmakologischen Eigenschaften bei der topischen und systemischen Therapie und Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, aber auch gegen Vitiligo, Ekzeme und Warzen, ferner gegen trockene Augen und andere Corneopathien sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen und durch Sonnenlichteinwirkung bedingten oder iatrogenen Hautschädigungen, z. B. durch Corticosteroide induzierter Atrophie, die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis-oder Streptokokkenzellwandinduzierten-Arthritis-Modell bestimmt werden. Die dermatologische Aktivität, beispielsweise für die Behandlung von Akne, kann u. a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben.

Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J. Am. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung kosmetische und therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels sowie ihre Verwendung zur Herstellung kosmetischer Präparationen.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions-oder Injektionslösungen sowie Pasten, Salben, Gele, Cremes, Lotionen, Puder, Lösungen oder Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Zur oralen Applikation geeignete Darreichungsformen sind beispielsweise Tabletten, Filmtabletten, Dragees, Kapseln, Pillen, Pulver, Lösungen oder Suspensionen oder Depotformen. Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Dragreehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt oder in Gelatinekapseln einkapselt.

Zweckmäßige übliche Bestandteile von kosmetischen und pharmazeutischen Zubereitungen für die topische Anwendung sind beispielsweise:

anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,

feste oder flüssige Ölkomponenten bzw. Fettstoffe mineralischer, pflanzlicher oder tierischer Herkunft, synthetische Esteröle, wie Triglyceridester und Isopropylmyristat,

hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, etherische Öle umd Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Herstellung der Ausgangsverbindungen

Beispiel A

1,4-Dimethoxy-2-formyl-5,6,7,8-tetrahydro-5,5,8,8-tetramethyl-naphthalin

92,9 g (0,37 mol) 1,4-Dimethoxy-5,6,7,8-tetrahydro-5,5,8,8-tetramethylnaphthalin und 52,5 g (0,37 mol) Hexamethylentetramin wurden in 350 ml Trifluoressigsäure 2 Std. unter Rückfluß erhitzt. Die Reaktionslösung wurde im Vakuum einkonzentriert, der Rückstand auf Eis gegossen, mit festem Natriumcarbonat neutralisiert und mit Ether extrahiert. Die mit Wasser gewaschenen und über Magnesiumsulfat getrockneten Extrakte lieferten nach Abdampfen des Lösungsmittels einen öligen Rückstand. Reinigung mittels Flash-Chromatographie lieferte 45 g der Titelverbindung, die aus Heptan umkristallisiert wurde. Schmp. 55-57°C.

Beispiel B

1,1,2,3,3-Pentamethyl-2,3-dihydro-5(1H)-indenyl-cyclopropylketon

Zu einer unter Kühlung bereiteten Suspension von 125 g (0,94 mol) wasserfreiem Aluminiumchlorid in 185 ml trockenem Methylenchlorid wurden 125 g (1,2 mol) Cyclopropylcarbonsäurechlorid und anschließend bei 0-5°C 158 g (0,84 mol) 1,1,2,3,3-Pentamethyl-2,3-dihydro-(1H)-indan, gelöst in 230 ml trockenem Methylenchlorid, zugetropft. Die Reaktionslösung rührte über Nacht bei 5°C, wurde auf Eis gegossen und mit Methylenchlorid extrahiert. Die Extrakte wurden mit Natriumcarbonatlösung und Wasser neutral gewaschen, über Natriumsulfat getrocknet und eingeengt. Der ölige Rückstand lieferte nach Destillation 159 g der Titelverbindung als farbloses Öl, $Kp_{0,4}$: 120-125°C, $n^{22}$: 1,5425.

Beispiel C

3,5-Di-tert.-butyl-phenylhydrazin

22 g (0,107 mol) 3,5-Di-tert.-butyl-anilin wurden in 20 ml Wasser bei 80°C mit 5,7 g (50 mmol) Hydroxylamin-O-sulfonsäure tropfenweise versetzt; nach Abkühlen wurde der Niederschlag abgesaugt und mit Toluol ausgekocht. Der Rückstand lieferte nach Behandlung mit 2 N Natronlauge, Extraktion mit Methylenchlorid und Abdampfen des Lösungsmittels ein Öl, das an Kieselgel im System n-Heptan/Essigester (10:1) chromatographiert wurde. Nach Abtrennung des unumgesetzten Ausgangsanilins wurde im System n-Heptan/Essigester (10:3) die Titelverbindung eluiert. Man erhielt 3,5 g eines Öls, das aus Ether mit etherischer Salzsäure ein farbloses Salz bildete, Schmp.: 196-199°C.

Herstellung der Endprodukte

Beispiel 1

1,4-Dimethoxy-5,6,7,8-tetrahydro-5,5,8,8-tetramethyl-napthyl-2-aldehyd-N-(4-carboxyphenyl)-hydrazon

8,3 g (30 mmol) 1,4-Dimethoxy-2-formyl-5,6,7,8-tetrahydro-5,5,8,8-tetramethyl-naphthalin (Beispiel A) und 4,6 g (30 mmol) Phenylhydrazin-4-carbonsäure wurden in 75 ml Tetrahydrofuran unter Rückfluß gerührt. Nach beendeter Reaktion wurde das Lösungsmittel abgedampft. Umkristallisieren des Rückstandes aus Methanol lieferte 8,5 g der Titelverbindung, Schmp.: 238-245°C.

Beispiel 2

1,1,2,3,3-Pentamethyl-2,3-dihydro-5(1H)-indenyl-cyclopropylketon-N-(4-carboxyphenyl)-hydrazon

7,7 g (30 mmol) 1,1,2,3,3-Pentamethyl-2,3-dihydro-5(1H)-indenyl-cyclopropylketon (Beispiel B) und 4,6 g (30 mmol) Phenylhydrazin-4-carbonsäure wurden in einem Gemisch aus 75 ml Tetrahydrofuran, 10 ml Ethanol und 2 ml Eisessig unter Rückfluß gerührt. Nach beendeter Reaktion wurde die Lösung eingedampft und der Rückstand aus Ethanol umkristallisiert. Man erhielt 3,9 g der Titelverbindung, Schmp.: 227-231°C.

Beispiel 3

1,2,3,4-Tetrahydro-1,1,4,4-tetramethyl-naphthalenyl-6-aldehyd-N-(4-carboxyphenyl)-hydrazon

5,0 g (23 mmol) 1,2,3,4-Tetrahydro-1,1,4,4-tetramethyl-naphthalenyl-6-aldehyd und 3,8 g (25 mmol) Phenylhydrazin-4-carbonsäure wurden in 50 ml Tetrahydrofuran 1 Std. unter Rückfluß gerührt. Nach Erkalten wurde die Reaktionslösung auf Heptan gegossen, der Niederschlag abfiltriert und aus Methanol umkristallisiert. Man erhielt 5,4 g der Titelverbindung, Schmp.: 260°C.

Beispiel 4

1,2,3,4-Tetrahydro-1,1,4,4-tetramethyl-naphthalenyl-6-methylketon-N-(4-carboxyphenyl)-hydrazon

5,0 g (22 mmol) 1,2,3,4-Tetrahydro-1,1,4,4-tetramethylnaphthalenyl-6-methylketon und 3,8 g (25 mmol)

Phenylhydrazin-4-carbonsäure wurden entsprechend Beispiel 3 umgesetzt und lieferten nach Umkristallisieren aus Ethanol 3,6 g der Titelverbindung, Schmp.: 250°C.

Beispiel 5

4-Methoxycarbonyl-benzaldehyd-N-(3,5-di-tert.-butyl-phenyl)-hydrazon

3,5 g (15 mmol) 3,5-Di-tert.-butyl-phenylhydrazin und 2,6 g (15 mmol) 4-Formyl-benzoesäuremethylester wurden in 150 ml Tetrahydrofuran/Ethanol (1:1) 1 Std. unter Rückfluß gerührt. Nach Abdampfen des Lösungsmittels wurde der Rückstand in der Siedehitze mit Ethanol digeriert, nach Erkalten abgesaugt und mit Ethanol nachgewaschen. Nach Trocknung im Vakuum bei Raumtemperatur erhielt man 3,2 g der Titelverbindung, Schm.: 215-220°C.

Beispiel 6

4-Carboxybenzaldehyd-N-(3,5-di-tert.-butyl-phenyl)hydrazon

2,2 g des in Beispiel 5 erhaltenen Esters wurden in 70 ml Ethanol/Dimethylsulfoxid/Wasser (3:3:1) mit 3 g Kaliumhydroxid 1 Std. zum Rückfluß erhitzt. Das Reaktionsgemisch wurde in 140 ml Eiswasser gegossen, mit 2 N Salzsäure auf pH 2 gestellt und der Niederschlag abgesaugt. Nach Umkristallisieren aus Methanol erhielt man 1,2 g der Titelverbindung, Schm.: 208-213°C.

Analog zu den Beispielen 1-6 wurden die in der nachfolgenden Tabelle aufgeführten Beispiele hergestellt:

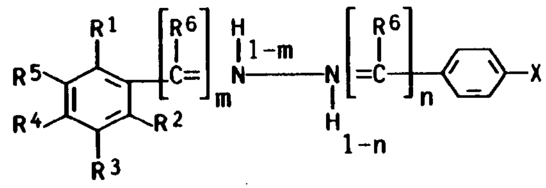

I,

| Nr. | $R^1$ | $R^2$ | $R^3$ | $R^4$ | $R^5$ | m | n | $R^6$ | X | Schmp. (°C) |
|---|---|---|---|---|---|---|---|---|---|---|
| 7 | H | H | H | $-C(CH_3)_2CH_2CH_2-C(CH_3)_2-$ | | 1 | 0 | H | $OCH_3$ | 142-146 |
| 8 | H | H | H | $-C(CH_3)_2CH_2CH_2-C(CH_3)_2-$ | | 1 | 0 | H | $NO_2$ | 227-231 |
| 9 | H | H | H | $-C(CH_3)_2CH_2CH_2-C(CH_3)_2-$ | | 1 | 0 | H | $SO_2CH_3$ | 228-233 |
| 10 | H | H | H | $-C(CH_3)_2-CH_2CO-C(CH_3)_2-$ | | 1 | 0 | $CH_3$ | $CO_2H$ | 275-279 |
| 11 | H | OH | $C_2H_5$ | H | $C_2H_5$ | 1 | 0 | $CH_3$ | $CO_2H$ | 239-241 |
| 12 | H | $CH_3$ | H | $-C(CH_3)_2-CH_2CH_2-C(CH_3)_2-$ | | 1 | 0 | H | $CO_2H$ | 216-220 |
| 13 | H | H | H | $-C(CH_3)_2-CH(CH_3)-C(CH_3)_2-$ | | 1 | 0 | $C_2H_5$ | $CO_2H$ | 266-269 |
| 14 | H | H | H | $-C(CH_3)_2-COCH_2-C(CH_3)_2-$ | | 1 | 0 | $CH_3$ | $CO_2H$ | 302-305 |
| 15 | H | H | H | $-C(CH_3)_2-CH(CH_3)-C(CH_3)_2-$ | | 1 | 0 | $CH_3$ | $CO_2H$ | 268-274 |
| 16 | H | H | H | $-NHCO-CH_2-C(CH_3)_2-$ | | 1 | 0 | $CH_3$ | $CO_2H$ | |
| 17 | H | $OCH_3$ | H | $-C(CH_3)_2-CH_2CH_2-C(CH_3)_2-$ | | 1 | 0 | $CH_3$ | $CO_2H$ | 230-234 |
| 18 | H | $CH_3$ | H | $-C(CH_3)_2-CH_2CH_2-C(CH_3)_2-$ | | 1 | 0 | $CH_3$ | $CO_2H$ | 275-278 |
| 19 | H | H | H | $-C(CH_3)_2-CH_2CH_2-C(CH_3)_2-$ | | 1 | 0 | 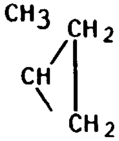 | $CO_2H$ | 243-245 |

Tabelle (Fortsetzung)

| Nr. | $R^1$ | $R^2$ | $R^3$ | $R^4$ | $R^5$ | m | n | $R^6$ | X | Schmp. (°C) |
|---|---|---|---|---|---|---|---|---|---|---|
| 20 | H | F | H | $-C(CH_3)_2-CH_2CH_2-C(CH_3)_2-$ | | 1 | 0 | $CH_3$ | $CO_2H$ | 256-260 |
| 21 | H | H | $CH_3$ | $-OCH_2CH_2-C(CH_3)_2-$ | | 1 | 0 | $CH_3$ | $CO_2H$ | |
| 22 | $OC_6H_{13}$ | $CH_3$ | H | $-C(CH_3)_2-CH_2CH_2-C(CH_3)_2-$ | | 1 | 0 | H | $CO_2H$ | 155-156 |
| 23 | H | H | H | $-CH_2CH_2CH_2C(CH_3)_2-$ | | 1 | 0 | $CH_3$ | $CO_2H$ | |
| 24 | $OCH_3$ | $CH_3$ | H | $-C(CH_3)_2-CH_2CH_2-C(CH_3)_2-$ | | 1 | 0 | H | $CO_2H$ | 220-223 |
| 25 | H | $OCH_3$ | H | $-C(CH_3)_2-CH_2CH_2-C(CH_3)_2-$ | | 1 | 0 | H | $CO_2H$ | 257-260 |
| 26 | $OCH_3$ | $OCH_3$ | H | $-C(CH_3)_2-CH_2CH_2-C(CH_3)_2-$ | | 1 | 0 | H | $SO_2CH_3$ | 92- 94 |
| 27 | H | F | H | $-C(CH_3)_2-CH_2CH_2-C(CH_3)_2-$ | | 1 | 0 | H | $CO_2H$ | 234-237 |
| 28 | H | H | $C(CH_3)_3$ | H | $C(CH_3)_3$ | 1 | 0 | H | $CO_2H$ | 259-262 |
| 29 | OH | $CH_3$ | H | $-C(CH_3)_2-CH_2CH_2-C(CH_3)_2-$ | | 1 | 0 | H | $CO_2H$ | 297-300 |
| 30 | H | H | $C(CH_3)_3$ | OH | $C(CH_3)_3$ | 1 | 0 | H | $CO_2H$ | 248-249 |
| 31 | H | H | H | $-C(CH_3)_2-CH_2CH(OH)-C(CH_3)_2-$ | | 1 | 0 | H | $CO_2H$ | 236-240 |
| 32 | H | H | H | $C(CH_3)_3$ | H | 1 | 0 | $C_2H_5$ | $CO_2H$ | 212-215 |
| 33 | H | H | H | $C(CH_3)_3$ | H | 1 | 0 | $CH_3$ | $CO_2H$ | 260-262 |
| 34 | H | H | $OC(CH_3)_3$ | H | H | 1 | 0 | $CH_3$ | $CO_2H$ | 214-217 |
| 35 | H | H | $CH(CH_3)_2$ | OH | $CH(CH_3)_2$ | 1 | 0 | $CH_3$ | $CO_2H$ | 217-220 |
| 36 | H | H | H | $C(CH_3)_2OCH_3$ | H | 1 | 0 | H | $CO_2H$ | 196-200 |
| 37 | H | H | H | $-C(CH_3)_2-CH_2CH_2-C(CH_3)_2-$ | | 1 | 0 | H | H | 80- 82 |
| 38 | H | H | H | $-C(CH_3)_2-CH_2CH_2-C(CH_3)_2-$ | | 1 | 0 | H | CN | |
| 39 | H | H | H | $-C(CH_3)_2-CH_2CH_2-C(CH_3)_2-$ | | 1 | 0 | H | $CO_2C_2H_5$ | 207 |
| 40 | H | H | H | $-C(CH_3)_2-CH_2CH_2-C(CH_3)_2-$ | | 1 | 0 | H | $CON(CH_3)_2$ | 223 |
| 41 | OAc | H | OAc | $-C(CH_3)_2-CH_2CH_2-C(CH_3)_2-$ | | 1 | 0 | H | $CO_2H$ | |
| 42 | OH | H | OH | $-C(CH_3)_2-CH_2CH_2-C(CH_3)_2-$ | | 1 | 0 | H | $CO_2H$ | |
| 43 | H | OAc | $C_2H_5$ | H | $C_2H_5$ | 1 | 0 | $CH_3$ | $CO_2H$ | |

Tabelle (Fortsetzung)

| Nr. | $R^1$ | $R^2$ | $R^3$ | $R^4$ | $R^5$ | m | n | $R^6$ | X | Schmp. (°C) |
|---|---|---|---|---|---|---|---|---|---|---|
| 44 | H | H | H | $-C(CH_3)_2-CH_2CH_2-C(CH_3)_2-$ | | 1 | 0 | H | $SO_3H$ | 280 (Zers.) |
| 45 | H | $CH_3$ | H | $-C(CH_3)_2-CH(CH_3)-CH_2-C(CH_3)_2-$ | | 1 | 0 | $CH_3$ | $CO_2H$ | |
| 46 | H | H | H | $-C(CH_3)_2-CH_2CH_2-C(CH_3)_2-$ | | 1 | 0 | $CH_3$ | $SO_2CH_3$ | 211-214 |
| 47 | H | H | H | $-C(CH_3)_2-CH_2CH_2-C(CH_3)_2-$ | | 1 | 0 | H | $SO_2C_2H_5$ | 177-181 |
| 48 | H | H | H | $-C(CH_3)_2-CH_2CH_2-C(CH_3)_3-$ | | 0 | 1 | H | $SO_2C_2H_5$ | 188-190 |
| 49 | H | H | H | $-C(CH_3)_2-CH_2CH_2-C(CH_3)_3-$ | | 0 | 1 | H | $CO_2CH_3$ | 141-143 |
| 50 | H | H | H | $-C(CH_3)_2-CH_2CH_2-C(CH_3)_3-$ | | 0 | 1 | H | $CO_2H$ | 187-189 |
| 51 | $OCH_3$ | H | $OCH_3$ | $-C(CH_3)_2-CH_2CH_2-C(CH_3)_2-$ | | 1 | 0 | H | $SO_2CH_3$ | 255-258 |
| 52 | H | $OCH_3$ | H | $-C(CH_3)_2-CH_2CH_2-C(CH_3)_2-$ | | 1 | 0 | H | $SO_2CH_3$ | 232-236 |
| 53 | H | F | H | $-C(CH_3)_2-CH_2CH_2-C(CH_3)_2-$ | | 1 | 0 | H | $SO_2CH_3$ | 244-247 |
| 54 | H | H | $C(CH_3)_3$ | H | $C(CH_3)_3$ | 1 | 0 | H | $SO_2CH_3$ | 190-192 |
| 55 | $OCH_3$ | $CH_3$ | H | $-C(CH_3)_2-CH_2CH_2-C(CH_3)_2-$ | | 1 | 0 | H | $SO_2CH_3$ | 224-227 |
| 56 | OH | $CH_3$ | H | $-C(CH_3)_2-CH_2CH_2-C(CH_3)_2-$ | | 1 | 0 | H | $SO_2CH_3$ | 331-335 |
| 57 | H | H | $C(CH_3)_3$ | OH | $C(CH_3)3$ | 1 | 0 | H | $SO_2CH_3$ | 206-209 |
| 58 | H | F | H | $-C(CH_3)_2-CH_2CH_2-C(CH_3)_2-$ | | 1 | 0 | $CH_3$ | $SO_2CH_3$ | 190-193 |
| 59 | H | H | H | $-C(CH_3)_2-CH_2CH_2-C(CH_3)_2-$ | | 1 | 0 | H | CONHOH | |
| 60 | H | H | H | $-C(CH_3)_2-CH_2CH_2-C(CH_3)_2-$ | | 1 | 0 | H | $CONHOCH_3$ | |
| 61 | H | H | H | $-C(CH_3)_2-CH_2CH_2-C(CH_3)_2-$ | | 1 | 0 | $CH_3$ | $CON(CH_3)OH$ | |
| 62 | H | H | $C(CH_3)_3$ | OH | $C(CH_3)_3$ | 1 | 0 | $CH_3$ | $SO_2CH_3$ | 233-236 |
| 63 | H | H | $C(CH_3)_3$ | OH | $C(CH_3)_3$ | 1 | 0 | $CH_3$ | $CO_2H$ | 249-250 (Zers.) |
| 64 | H | H | H | $-C(CH_3)_2-CH_2CH_2-C(CH_3)_2-$ | | 1 | 0 | H | $PO(OCH_3)_2$ | |
| 65 | H | H | $C(CH_3)_3$ | OH | $C(CH_3)_3$ | 1 | 0 | $CH_3$ | $CO_2CH_3$ | 180-182 |
| 66 | H | H | $C(CH_3)_3$ | OH | $C(CH_3)_3$ | 1 | 0 | $CH_3$ | $SO_2C_2H_5$ | 163 |
| 67 | H | H | $C(CH_3)_3$ | OH | $C(CH_3)_3$ | 1 | 0 | $CH_3$ | $SO_2CH(CH_3)_2$ | 120 |

Tabelle (Fortsetzung)

| Nr. | $R^1$ | $R^2$ | $R^3$ | $R^4$ | $R^5$ | m | n | $R^6$ | X | Schmp. (°C) |
|---|---|---|---|---|---|---|---|---|---|---|
| 68 | H | H | $C(CH_3)_3$ | OH | $C(CH_3)_3$ | 1 | 0 | $CH_3$ | $CONH_2$ | 212 |
| 69 | H | H | $C(CH_3)_3$ | OH | $C(CH_3)_3$ | 1 | 0 | $CH_3$ | $CO_2C_2H_5$ | 129-132 |
| 70 | H | H | $C(CH_3)_3$ | OH | $C(CH_3)_3$ | 1 | 0 | $CH_3$ | $CO_2C_6H_5$ | 209-210 |
| 71 | $OCH_3$ | $OCH_3$ | H | $-C(CH_3)_2-CH_2CH_2-C(CH_3)_2$ | | 1 | 0 | H | $CO_2H$ | 171-174 |
| 72 | H | H | H | $-C(CH_3)_2-CH_2CH_2-C(CH_3)_2$ | | 1 | 0 | $CH_3$ | $SO_2CH(CH_3)_2$ | 156-159 |
| 73 | H | H | H | $-C(CH_3)_3-CH_2CH_2-C(CH_3)_3$ | | 1 | 0 | H | $SO_2CH(CH_3)_2$ | 205-206 |
| 74 | H | H | $C(CH_3)_3$ | H | $C(CH_3)_3$ | 1 | 0 | H | $SO_2C_2H_5$ | 222-224 |
| 75 | H | H | $C(CH_3)_3$ | H | $C(CH_3)_3$ | 1 | 0 | H | $SO_2CH(CH_3)_2$ | 203-205 |
| 76 | H | H | $C(CH_3)_3$ | OH | $C(CH_3)_3$ | 1 | 0 | $CH_3$ | $SO_2$ 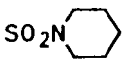 | |
| 77 | H | H | $C(CH_3)_3$ | OH | $C(CH_3)_3$ | 1 | 0 | $CH_3$ | $SO_2$ 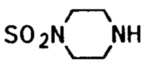 | |
| 78 | H | H | $C(CH_3)_3$ | OH | $C(CH_3)_3$ | 1 | 0 | $CH_3$ | $SO_2$ 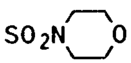 | |
| 79 | H | H | $C(CH_3)_3$ | OH | $C(CH_3)_3$ | 1 | 0 | $CH_3$ | CO 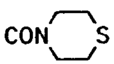 | |
| 80 | $OC_3H_7$ | $CH_3$ | H | $-C(CH_3)_2CH_2-C(CH_3)_2-$ | | 1 | 0 | H | $CO_2H$ | 199-200 |
| 81 | $OC_2H_5$ | $CH_3$ | H | $-C(CH_3)_2-CH_2CH_2-C(CH_3)_2-$ | | 1 | 0 | H | $CO_2H$ | 214-216 |
| 82 | H | H | H | $-C(CH_3)_2-CH_2CH_2-C(CH_3)_2-$ | | 1 | 0 | H | $SOCH_3$ | |
| 83 | H | H | $C(CH_3)_3$ | OH | $C(CH_3)_3$ | 1 | 0 | H | $SO_3H$ | |
| 84 | H | H | $C(CH_3)_3$ | H | $C(CH_3)_3$ | 1 | 0 | $CH_3$ | $SO_3H$ | |

Beschreibung zu Beispiel 46

1,2,3,4-Tetrahydro-1,1,4,4-tetramethyl-naphthalenyl-6-methylketon-N-(methylsulfonylphenyl)hydrazon (anti-Isomer)

4,7 g (20 mmol) 1,2,3,4-Tetrahydro-1,1,4,4-tetramethylnaphthalenyl-6-methylketon und 4,0 g (20 mmol) 4-Methylsulfonylphenylhydrazin wurden in 60 ml Ethanol unter Rückfluß erhitzt. Nach beendeter Reaktion wurde der Niederschlag abgesaugt, mit Ethanol nachgewaschen und bei 50°C in Vakuum getrocknet. Man erhielt 7,0 g der Titelverbindung, Schmp. 211-214°C.

Beispiel 46a

1,2,3,4-Tetrahydro-1,1,4,4-tetramethylnaphthalenyl-6-methylketon-N-(4-methylsulfonylphenyl)-hydrazon (syn.-Isomer)

Die Mutterlauge der voranstehenden Beispielverbindung wurde zur Trockene eingedampft und der Rückstand an Kieselgel im System Heptan/Essigsäureethylester (99 : 1 bis 80 : 20) chromatographiert. Nach Eindampfen des entsprechenden Eluates erhielt man 0,2 g der Titelverbindung, Schmp. 216-218°C.

Beispiele für pharmazeutische Zubereitungen:

Beispiel I

Tablette mit 250 mg Wirkstoff

Zusammensetzung für 1000 Tabletten:

| | |
|---|---|
| Wirkstoff des Beispiels Nr. 2: | 250 g |
| Kartoffelstärke: | 100 g |
| Milchzucker: | 50 g |
| Gelatinelösung 4 %: | 45 g |
| Talkum: | 10 g |

Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel II

Creme aus 0,1 % Wirkstoff

| | |
|---|---|
| Wirkstoff des Beispiels Nr. 7: | 0,1 g |
| Glycerinmonostearat: | 10,0 g |
| Cetylalkohol: | 4,0 g |
| Polyethylenglykol-400-stearat: | 10,0 g |
| Polyethylenglykol-sorbitanmonostearat: | 10,0 g |
| Propylenglykol: | 6,0 g |
| p-Hydroxybenzoesäuremethylester: | 0,2 g |
| Entmineralisiertes Wasser: | ad 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel III

Puder mit 0,1 % Wirkstoff

| | |
|---|---|
| Wirkstoff des Beispiels Nr. 4: | 0,1 g |
| Zinkoxid: | 10,0 g |
| Magnesiumoxid: | 10,0 g |
| Hochdisperses Siliciumdioxid: | 2,5 g |
| Magnesiumstearat: | 1,0 g |
| Talkum: | 76,4 g |

Herstellung

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

## Patentansprüche

**Patentansprüche für folgende Vertragsstaaten : AT, BE, CH, DE, DK, FR, GB, IT, LI, NL, SE**

1. Phenylhydrazone der Formel I,

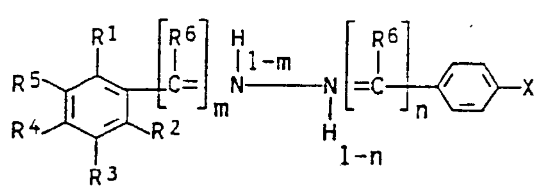

I,

in der bedeuten:

$R^1$, $R^2$ und $R^3$ unabhängig voneinander ein Wasserstoff-oder Halogenatom, eine $C_1$-$C_4$-Alkylgruppe, eine Hydroxyl-oder $C_1$-$C_4$-Alkoxygruppe oder den Acetoxyrest,

$R^4$ ein Wasserstoffatom, eine Hydroxylgruppe oder einen tert-Butyl- oder $C_1$-$C_6$-Alkoxy- oder $C_2$-$C_6$-Alkoxyalkylrest,

$R^5$ ein Wasserstoffatom oder eine $C_1$-$C_4$-Alkylgruppe, oder

$R^4$ und $R^5$ unter Bildung eines Zyklus gemeinsam eine $-C(CH_3)_2-A-C(CH_3)_2$-Gruppe (mit A in der Bedeutung von $-CH_2CH_2-$, $-CH(CH_3)-$, $-CH_2C(O)-$ oder $-CH_2CHOH-$) oder eine $-(CH_2)_3C(CH_3)_2-$, eine $-OCH_2CH_2C(CH_3)_2-$, eine $-C(CH_3)_2CH(CH_3)CH_2C(CH_3)_2-$oder eine $-NHC(O)CH_2C(CH_3)_2$-Gruppe, wobei, wenn $R^1$ bis $R^3$ Wasserstoff sind, $R^4$ und $R^5$ gemeinsam einen Zyklus der genannten Art bilden oder $R^4$ einen verzweigten $C_4$-$C_6$-Alkoxy- oder Alkoxyalkylrest darstellt,

$R^6$ ein Wasserstoffatom, den Methyl-, Ethyl- oder Cyclopropylrest,

m und n verschieden voneinander 0 oder 1,

X ein Wasserstoffatom, eine Nitro-, Methoxy- oder Nitrilgruppe, den Sulfonsäurerest oder die Reste $-CONR^7OR^7$, $-CO_2R^7$, $-PO(OR^8)_2$, $S(O)_n$ $R^8$ (mit n = 0, 1 oder 2), $-SO_2-NR^9R^{10}$ oder $-CONR^9R^{10}$, worin $R^7$ in der Bedeutung von Wasserstoff, einer $C_1$-$C_3$-Alkyl- oder einer Phenylgruppe, die gegebenenfalls durch ein bis zwei Amino-, $C_1$-$C_4$-Acylamino-, -Alkyl- oder Alkoxygruppen substituiert ist, und $R^8$ in der Bedeutung einer $C_1$-$C_3$-Alkylgruppe steht, $R^9$ und $R^{10}$ unabhängig voneinander Wasserstoff oder eine $C_1$-$C_4$-Alkylgruppe bedeuten oder gemeinsam einen Piperidin-, Piperazin-, Morpholin- oder Thiomorpholinring bilden, wobei, wenn X ein Wasserstoffatom, eine Methoxy-, Nitril- oder eine Nitrogruppe oder wenn X = Carboxyl und $R^1$ oder $R^2$ = Hydroxyl ist, $R^4$ und $R^5$ gemeinsam einen Zyklus der obengenannten Art bilden oder $R^3$ und $R^5$ jeweils i-Propyl oder iso- oder tert-Butyl darstellen, und, wenn m=0 ist, $R^4$ keine Hydroxylgruppe darstellt,

sowie deren physiologisch verträgliche Salze.

2. Verfahren zur Herstellung der Phenylhydrazone der Formel I gemäß Anspruch 1, dadurch gekennzeichnet, daß man

a) Carbonylverbindungen der Formel IIa

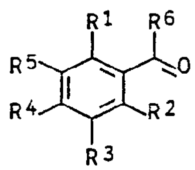

IIa,

in der $R^1$ bis $R^6$ die in den Ansprüchen 1 bis 6 angegebenen Bedeutungen haben, mit Phenylhydrazinen der Formel IIIa

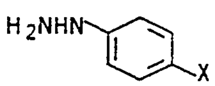

IIIa,

in der X die in den Ansprüchen 1 bis 6 angegebene Bedeutung hat, umsetzt, oder

b) Carbonylverbindungen der Formel IIb

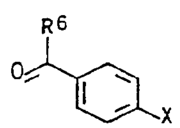

IIb,

in der $R^6$ und X die genannten Bedeutungen haben, mit Phenylhydrazinen der Formel IIIb

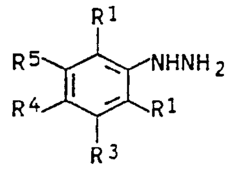

IIb,

in der $R^1$ bis $R^5$ die genannten Bedeutungen haben, umsetzt.

3. Arzneimittel zur topischen Anwendung, das neben üblichen galenischen Hilfsmitteln 0,001 bis 1 Gew.% eines Phenylhydrazons der Formel I gemäß Anspruch 1 enthält.

4. Arzneimittel zur systemischen Anwendung, das neben üblichen galenischen Hilfsmitteln als Wirkstoff pro Einzeldosis 0,1 bis 250 mg eines Phenylhydrazons der Formel I nach Anspruch 1 enthält.

5. Kosmetische Zubereitung, die neben üblichen kosmetischen Hilfsmitteln 0,001 bis 1 Gew.% eines Phenylhydrazons der Formel I nach Anspruch 1 enthält.

6. Arzneimittel nach Anspruch 3 zur Behandlung von Akne oder Psoriasis oder anderen mit pathologischer Verhornung einhergehenden dermatologischen Erkrankungen sowie von Ekzemen, Warzen und Vitiligo.

7. Arzneimittel nach Anspruch 3 zur Behandlung von durch UV-Licht oder iatrogen bedingten Schädigungen der Haut.

8. Arzneimittel nach Anspruch 3 zur Behandlung von trockenen Augen und anderen Corneopathien.

9. Arzneimittel nach Anspruch 3 oder 4 zur Behandlung von Präkanzerosen und Tumoren.

10. Arzneimittel nach Anspruch 3 oder 4 zur Behandlung von rheumatischen und arthritischen Erkrankungen.

**Patentansprüche für folgende Verstragsstaaten : ES, GR**

1. Verfahren zur Herstellung von Phenylhydrazonen der Formel I,

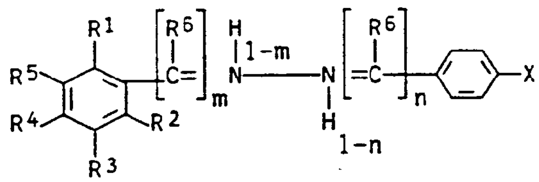

I,

in der bedeuten:

$R^1$, $R^2$ und $R^3$ unabhängig voneinander ein Wasserstoff-oder Halogenatom, eine $C_1$-$C_4$-Alkylgruppe, eine Hydroxyl-oder $C_1$-$C_4$-Alkoxygruppe oder den Acetoxyrest,

$R^4$ ein Wasserstoffatom, eine Hydroxylgruppe oder einen tert-Butyl- oder $C_1$-$C_6$-Alkoxy- oder $C_2$-$C_6$-Alkoxyalkylrest,

$R^5$ ein Wasserstoffatom oder eine $C_1$-$C_4$-Alkylgruppe, oder

$R^4$ und $R^5$ unter Bildung eines Zyklus gemeinsam eine $-C(CH_3)_2$-A-$C(CH_3)_2$-Gruppe (mit A in der Bedeutung von $-CH_2CH_2-$, $-CH(CH_3)-$, $-CH_2C(O)-$ oder $-CH_2CHOH-$) oder eine $-(CH_2)_3C(CH_3)_2-$, eine $-OCH_2CH_2C(CH_3)_2-$, eine $-C(CH_3)_2CH(CH_3)CH_2C(CH_3)_2$-oder eine $-NHC(O)CH_2C(CH_3)_2$-Gruppe, wobei, wenn $R^1$ bis $R^3$ Wasserstoff sind, $R^4$ und $R^5$ gemeinsam einen Zyklus der genannten Art bilden oder $R^4$ einen verzweigten $C_4$-$C_6$-Alkoxy- oder Alkoxyalkylrest darstellt,

$R^6$ ein Wasserstoffatom, den Methyl-, Ethyl- oder Cyclopropylrest,

m und n verschieden voneinander 0 oder 1,

X ein Wasserstoffatom, eine Nitro-, Methoxy- oder Nitrilgruppe, den Sulfonsäurerest oder die Reste $-CONR^7OR^7$, $-CO_2R^7$, $-PO(OR^8)_2$, $S(O)_n$ $R^8$ (mit n = 0, 1 oder 2), $-SO_2-NR^9R^{10}$ oder $-CONR^9R^{10}$, worin $R^7$ in der Bedeutung von Wasserstoff, einer $C_1$-$C_3$-Alkyl- oder einer Phenylgruppe, die gegebenenfalls durch ein bis zwei Amino-, $C_1$-$C_4$-Acylamino-, -Alkyl- oder Alkoxygruppen substituiert ist, und $R^8$ in der Bedeutung einer $C_1$-$C_3$-Alkylgruppe steht, $R^9$ und $R^{10}$ unabhängig voneinander Wasserstoff oder eine $C_1$-$C_4$-

Alkylgruppe bedeuten oder gemeinsam einen Piperidin-, Piperazin-, Morpholin- oder Thiomorpholinring bilden, wobei, wenn X ein Wasserstoffatom, eine Methoxy-, Nitril- oder eine Nitrogruppe oder wenn X = Carboxyl und $R^1$ oder $R^2$ = Hydroxyl ist, $R^4$ und $R^5$ gemeinsam einen Zyklus der obengenannten Art bilden oder $R^3$ und $R^5$ jeweils i-Propyl oder iso- oder tert-Butyl darstellen, und, wenn m=0 ist, $R^4$ keine Hydroxylgruppe darstellt,

sowie von deren physiologisch verträglichen Salzen, dadurch gekennzeichnet, daß man

a) Carbonylverbindungen der Formel IIa

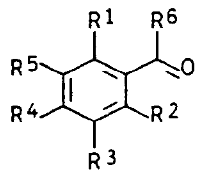

IIa,

in der $R^1$ bis $R^6$ die in den Ansprüchen 1 bis 6 angegebenen Bedeutungen haben, mit Phenylhydrazinen der Formel IIIa

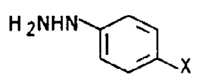

IIIa,

in der X die in den Ansprüchen 1 bis 6 angegebene Bedeutung hat, umsetzt, oder

b) Carbonylverbindungen der Formel IIb

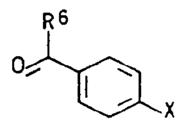

IIb,

in der $R^6$ und X die genannten Bedeutungen haben, mit Phenylhydrazinen der Formel IIIb

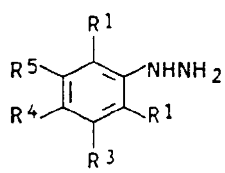

IIIb,

in der $R^1$ bis $R^5$ die genannten Bedeutungen haben, umsetzt,

und das Reaktionsprodukt ggf. mit einer physiologisch verträglichen Säure umsetzt.

**2.** Verfahren zur Herstellung eines Arzneimittels zur topischen Anwendung, dadurch gekennzeichnet, daß man übliche galenische Hilfsmittel mit 0,001 bis 1 Gew.% eines Phenylhydrazons der Formel I gemäß Anspruch 1 mischt.

**3.** Verfahren zur Herstellung eines Arzneimittels zur systemischen Anwendung, dadurch gekennzeichnet, daß man übliche galenische Hilfsmittel mit 0,1 bis 250 mg pro Einzeldosis eines Phenylhydrazons der Formel I nach Anspruch 1 mischt.

**4.** Verfahren zur Herstellung einer kosmetischen Zubereitung, dadurch gekennzeichnet, daß man übliche kosmetische Hilfsmittel mit 0,001 bis 1 Gew.% eines Phenylhydrazons der Formel I nach Anspruch 1 mischt.

**5.** Verfahren nach Anspruch 2 zur Herstellung eines Arzneimittels zur Behandlung von Akne oder Psoriasis oder anderen mit pathologischer Verhornung einhergehenden dermatologischen Erkrankungen sowie von Ekzemen, Warzen und Vitiligo.

6. Verfahren nach Anspruch 2 zur Herstellung eines Arzneimittels zur Behandlung von durch UV-Licht oder iatrogen bedingten Schädigungen der Haut.

7. Verfahren nach Anspruch 2 zur Herstellung eines Arzneimittels zur Behandlung von trockenen Augen und anderen Corneopathien.

8. Verfahren nach Anspruch 2 oder 3 zur Herstellung eines Arzneimittels zur Behandlung von Präkanzerosen und Tumoren.

9. Verfahren nach Anspruch 2 oder 3 zur Herstellung eines Arzneimittels zur Behandlung von rheumatischen und arthritischen Erkrankungen.

10. Kosmetische Zubereitung, die neben üblichen kosmetischen Hilfsmitteln 0,001 bis 1 Gew.-% eines Phenylhydrazons der Formel I nach Anspruch 1 enthält.

## Claims

**Claims for the following Contracting States : AT, BE, CH, DE, DK, FR, GB, IT, LI, NL, SE**

1. A phenylhydrazone of the formula I:

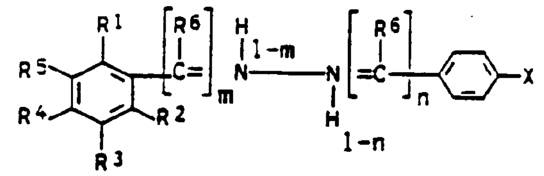

I,

in which $R^1$, $R^2$, and $R^3$ are independently of one another hydrogen, halogen, $C_1$-$C_4$-alkyl, hydroxyl, $C_1$-$C_4$-alkoxy or acetoxy, $R^4$ is hydrogen, hydroxyl, tert. -butyl, $C_1$-$C_6$-alkoxy or $C_2$-$C_6$-alkoxyalkyl, $R^5$ is hydrogen or $C_1$-$C_4$-alkyl, or $R^4$ and $R^5$ together form a ring which is $-C(CH_3)_2-A-C(CH_3)_2-$ (where A is $-CH_2CH_2-$, $-CH(CH_3)-$, $-CH_2C(O)-$ or $-CH_2CHOH-$) or is $-(CH_2)_3C(CH_3)_2-$, $-OCH_2CH_2C(CH_3)_2-$, $-C(CH_3)_2CH(CH_3)CH_2C(CH_3)_2-$ or $-NHC(O)CH_2C(CH_3)_2-$, where $R^4$ and $R^5$ together form a ring of the type specified or $R^4$ is branched $C_4$-$C_6$-alkoxy or branched $C_4$-$C_6$-alkoxyalkyl when $R^1$, $R^2$ and $R^3$ are each hydrogen, $R^6$ is hydrogen, methyl, ethyl or cyclopropyl, m and n are different and denote 0 or 1, X is hydrogen, nitro, methoxy or nitrile, a sulfonic acid radical or$CONR^7OR^7$, $-CO_2R^7$, $-PO(OR^8)_2$, $S(O)_nR^8$ (where n is 0, 1 or 2 ), $-SO_2-NR^9R^{10}$ or $-CONR^9R^{10}$, where $R^7$ is hydrogen, $C_1$-$C_3$-alkyl or phenyl, which may or may not be substituted by one or two amino, $C_1$-$C_4$-acylamino, $C_1$-$C_4$-alkyl or $C_1$-$C_4$-alkoxy groups, $R^8$ is $C_1$-$C_3$-alkyl and $R^9$ and $R^{10}$ are independently of each other hydrogen or $C_1$-$C_4$-alkyl or together form a piperidine, piperazine, morpholine or thiomorpholine ring, where $R^4$ and $R^5$ together form a ring of the type specified or $R^3$ and $R^5$ are each isopropyl, isobutyl or tert.-butyl when X is hydrogen, methoxy, nitrile or nitro or when X is carboxyl and $R^1$ or $R^2$ is hydroxyl, and $R^4$ is not hydroxyl when m is 0, and their physiologically tolerated salts.

2. A process for the preparation of a phenylhydrazone of the formula I as claimed in claim 1 which comprises a) reacting carbonyl compounds of the formula IIa

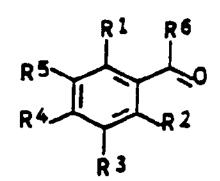

IIa,

in which $R^1$ and $R^6$ have the meanings stated in claim 1, with phenylhydrazines of the formula IIIa:

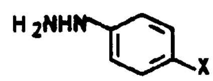

IIIa,

in which X has the meaning stated in claim 1, or
b) reacting carbonyl compounds of formula IIb:

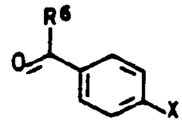

IIb,

in which $R^6$ and X have the meanings stated, with phenylhydrazines of the formula IIIb:

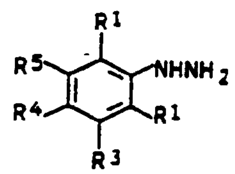

IIIb,

in which $R^1$ to $R^5$ have the meanings stated.

3. A drug for topical use containing, in addition to the customary pharmaceutical aids from 0.001 to 1% by weight of a phenylhydrazone of the formula I as claimed in claim 1.

4. A drug for systemic use containing, in addition to customary pharmaceutical aids, from 0.1 to 250 mg of a phenylhydrazone of the formula I as claimed in claim 1, as active ingredient, per individual dose.

5. A cosmetic formulation containing, in addition to customary cosmetic aids, from 0.001 to 1 % by weight of a phenylhydrazone of the formula as claimed in claim 1.

6. A drug as claimed in claim 3 for the treatment of acne or psoriasis or other dermatological diseases associated with pathological hornification, and eczema, warts and vitiligo.

7. A drug as claimed in claim 3 for the treatment of skin damage caused by U.V. light or iatrogenically.

8. A drug as claimed in claim 3 for the treatment of dry eyes or other corneopathies.

9. A drug as claimed in claim 3 or 4 for the treatment of precancerous stages and tumors.

10. A drug as claimed in claim 3 or 4 for the treatment of rheumatic and arthritic diseases.

**Claims for the following Contracting States : ES, GR**

1. A process for the preparation of a phenylhydrazone of the formula I:

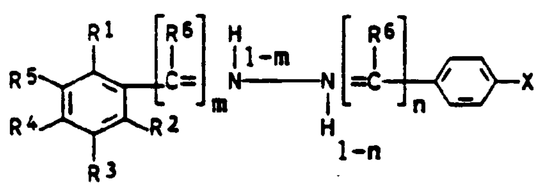

I,

in which $R^1$, $R^2$, and $R^3$ are independently of one another hydrogen, halogen, $C_1$-$C_4$-alkyl, hydroxyl, $C_1$-$C_4$-alkoxy or acetoxy, $R^4$ is hydrogen, hydroxyl, tert. -butyl $C_1$-$C_6$-alkoxy or $C_2$-$C_6$-alkoxyalkyl, $R^5$ is hydrogen or $C_1$-$C_4$-alkyl, or $R^4$ and $R^5$ together form a ring which is $-C(CH_3)_2-A-C(CH_3)_2-$(where A is $-CH_2CH_2-$,

$-CH(CH_3)-$, $-CH_2C(O)-$ or $-CH_2CHOH-$) or is $-(CH_2)_3C(CH_3)_2-$, $-OCH_2CH_2C(CH_3)_2-$, $-C(CH_3)_2CH(CH_3)CH_2C(CH_3)_2-$ or $-NHC(O)CH_2C(CH_3)_2-$, where $R^4$ and $R^5$ together form a ring of the type specified or $R^4$ is branched $C_4$-$C_6$-alkoxy or alkoxyalkyl when $R^1$, $R^2$ and $R^3$ are each hydrogen, $R^6$ is hydrogen, methyl, ethyl or cyclopropyl, m and n are different and denote 0 or 1, X is hydrogen, nitro, methoxy or nitrile, a sulfonic acid radical or $-CONR^7OR^7$, $-CO_2R^7$, $-PO(OR^8)_2$, $S(O)_nR^8$ (where n is 0, 1 or 2), - $SO_2$-$NR^9R^{10}$ or $-CONR^9R^{10}$, where $R^7$ is hydrogen, $C_1$-$C_3$-alkyl or phenyl, which may or may not be substituted by one or two amino, $C_1$-$C_4$-acylamino, $C_1$-$C_4$-alkyl or alkoxy groups, $R^8$ is $C_1$-$C_3$-alkyl and $R^9$ and $R^{10}$ are independently of each other hydrogen or $C_1$-$C_4$-alkyl or together form a piperidine, piperazine, morpholine or thiomorpholine ring, where $R^4$ and $R^5$ together form a ring of the type specified or $R^3$ and $R^5$ are each, methoxy, nitrile or nitro or when X is carboxyl and $R^1$ or $R^2$ is hydroxyl, and $R^4$ is not hydroxyl when m is 0, isopropyl, isobutyl or tert.-butyl when X is hydrogen and their physiologically tolerated salts which comprises

a) reacting carbonyl compounds of the formula IIa

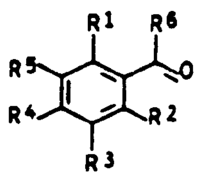

IIa,

in which $R^1$ and $R^6$ have the meanings stated in claim 1, with phenylhydrazines of the formula IIIa:

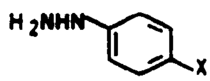

IIIa,

in which X has the meaning stated in claim 1, or

b) reacting carbonyl compounds of formula IIb:

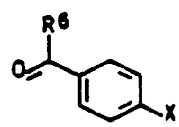

IIb,

in which $R^6$ and X have the meanings stated, with phenylhydrazines of the formula IIIb:

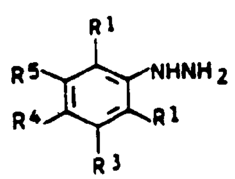

IIIb,

in which $R^1$ to $R^5$ have the meanings stated, and reacting the reaction product if desired with a physiologically tolerated acid.

**2.** A process for the preparation of a drug for topical use, which comprises mixing customary pharmaceutical aids with from 0.001 to 1% by weight of a phenylhydrazone of the formula I as claimed in claim 1.

**3.** A process for the preparation of a drug for systemic use, which comprises mixing customary pharmaceutical aids with from 0.1 to 250 mg per individual dose of a phenylhydrazone of the formula I as claimed in claim 1.

**4.** A process for the preparation of a cosmetic formulation, which comprises mixing customary cosmetic aids with from 0.001 to 1% by weight of a phenylhydrazone of the formula I as claimed in claim 1.

**5.** A process as claimed in claim 2 for the preparation of a drug for the treatment of acne or psoriasis or other

dermatological diseases associated with pathological hornification, and eczema, warts and vitiligo.

6. A process as claimed in claim 2 for the preparation of a drug for the treatment of skin damage caused by U.V. light or iatrogenically.

7. A process as claimed in claim 2 for the preparation of a drug for the treatment of dry eyes or other corneopathies.

8. A process as claimed in claim 2 or 3 a drug for the treatment of precancerous stages and tumors.

9. A process as claimed in claim 2 or 3 a drug for the treatment of rheumatic and arthritic diseases.

10. A cosmetic formulation which, in addition to customary cosmetic aids, contains from 0.001 to 1% by

## Revendications

### Revendications pour les Etats contractants suivants : AT, BE, CH, DE, DK, FR, GB, IT, LI, NL, SE

1. Phénylhydrazones de formule I

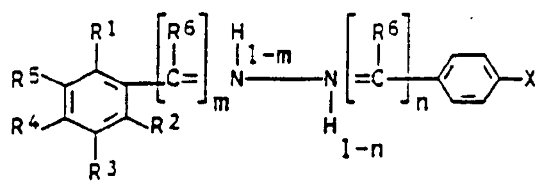

I,

dans laquelle représentent :

R¹, R² et R³, indépendamment les uns des autres, un atome d'hydrogène ou d'halogène, un groupe alkyle en $C_1$-$C_4$, un groupe hydroxyle ou alcoxy en $C_1$-$C_4$ ou le reste acétoxy.

$R^4$ un atome d'hydrogène, un groupe hydroxyle ou un reste tert-butyle ou alcoxy en $C_1$-$C_6$ ou alcoxyalkyle en $C_2$-$C_6$,

$R^5$ un atome d'hydrogène ou un groupe alkyle en $C_1$-$C_4$, ou $R^4$ et $R^5$ ensemble, en formant un cycle, un groupe $-C(CH_3)_2$-A-$C(CH_3)_2$ (avec A = $-CH_2CH_2-$, $-CH(CH_3)-$, $-CH_2C(O)-$ ou $-CH_2CHOH-$) ou un groupe $-(CH_2)_3C(CH_3)_2-$, un groupe $-OCH_2CH_2C(CH_3)_2-$, un groupe - $C(CH_3)_2CH(CH_3)(CH_2C(CH_3)_2$- ou un groupe - $NHC(O)CH_2C(CH_3)_2-$, $R^4$ et $R^5$, lorsque $R^1$ à $R^3$ sont de l'hydrogène, formant ensemble un cycle du type cité ou $R^4$ représentant un reste alcoxy en $C_4$-$C_6$ ou alcoxyalkyle ramifié,

$R^6$ un atome d'hydrogène, le reste méthyle, éthyle ou cyclopropyle,

m et n, différents l'un de l'autre, 0 ou 1,

X un atome d'hydrogène, un groupe nitro, méthoxy ou nitrile, le reste acide sulfonique ou les restes - $CONR^7OR^7$, $CO_2R^7$, $-PO(OR^8)_2$, $S(O)_nR^8$ (avec n = 0, 1 ou 2), $-SO_2-NR^9R^{10}$ ou $-CONR^9R^{10}$ où $R^7$ représente hydrogène, un groupe alkyle en $C_1$-$C_3$ ou un groupe phényle, éventuellement substitué par un ou deux groupes amino, acylamino en $C_1$-$C_4$, alkyle ou alcoxy en $C_1$-$C_4$, et $R^8$ représente un groupe alkyle an $C_1$-$C_3$, $R^8$ et $R^9$, indépendamment l'un de l'autre, représentant hydrogène ou un groupe alkyle en $C_1$-$C_4$ ou formant ensemble un cycle pipéridine, pipérazine, morpholine ou thiomorpholine,

phénylhydrazones dans lesquelles, lorsque X est un atome d'hydrogène, un groupe méthoxy, nitrile ou nitro ou lorsque X = carboxyle et $R^1$ ou $R^2$ = hydroxyle, $R^4$ et $R^5$ forment, ensemble, un cycle du type cité plus haut ou $R^3$ et $R^5$ représentent, chacun, i-propyle ou iso- ou tert-butyle et si m = 0, $R^4$ ne représente pas le groupe hydroxyle, ainsi que leurs sels acceptables physiologiquement.

2. Procédé de préparation des phénylhydrazones de formule I, selon la revendication 1, caractérisé par le fait que l'on met à réagir :

a) des composés carbonyles de formule IIa

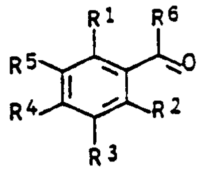

IIa,

dans laquelle $R^1$ à $R^6$ ont les significations données dans les revendications 1 à 6, avec des phénylhydrazines de formule IIIa

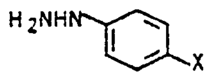

IIIa,

dans laquelle X a les significations données dans les revendications 1 à 6, ou
b) des composés carbonyles de formule IIb

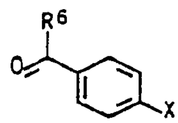

IIb,

dans laquelle $R^6$ et X ont les significations données plus haut, avec des phénylhydrazines de formule IIIb

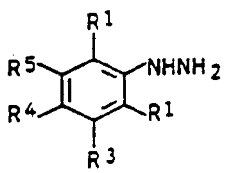

IIIb,

dans laquelle $R^1$ à $R^5$ ont les significations données plus haut.

3. Médicament pour l'administration topique, contenant, outre les auxiliaires galéniques usuels, 0,001 à 1 % en poids d'une phénylhydrazone de formule I, selon la revendication 1.

4. Médicament pour l'administration systémique, contenant comme principe actif, par dose unitaire, outre les auxiliaires galéniques usuels 0,1 à 250 mg d'une phénylhydrazone de formule I, selon la revendication 1.

5. Préparation cosmétique contenant, outre les auxiliaires cosmétiques usuels, 0,001 à 1 % en poids d'une phénylhydrazone de formule I, selon la revendication 1.

6. Médicament selon la revendication 3, pour le traitement de l'acné ou du psoriasis ou d'autres maladies dermatologiques accompagnées de la formation de callosités pathologiques, ainsi que d'eczémas, verrues et vitiligo.

7. Médicament selon la revendication 3, pour le traitement de lésions iatrogènes de la peau ou de lésions causées par la lumière UV.

8. Médicament selon la revendication 3, pour le traitement d'yeux secs et d'autres cornéopathies.

9. Médicament selon l'une des revendications 3 ou 4, pour le traitement de précancéroses et de tumeurs.

10. Médicament selon l'une des revendications 3 ou 4 pour le traitement des rhumatismes et de l'arthrite.

**Revendications pour les Etats contractants suivants : ES, GR**

1. Procédé de préparation de phénylhydrazones de formule I

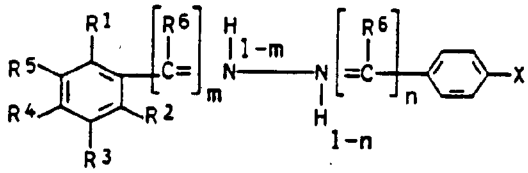

I,

dans laquelle représentent :

$R^1$, $R^2$ et $R^3$, indépendamment les uns des autres, un atome d'hydrogène ou d'halogène, un groupe alkyle en $C_1$-$C_4$, un groupe hydroxyle ou alcoxy en $C_1$-$C_4$ ou le reste acétoxy.

$R^4$ un atome d'hydrogène, un groupe hydroxyle ou un reste tert-butyle ou alcoxy en $C_1$-$C_6$ ou alcoxyalkyle en $C_2$-$C_6$,

$R^5$ un atome d'hydrogène ou un groupe alkyle en $C_1$-$C_4$, ou $R^4$ et $R^5$ ensemble, en formant un cycle, un groupe $-C(CH_3)_2$-A-$C(CH_3)_2$ (avec A = $-CH_2CH_2-$, $-CH(CH_3)-$, $-CH_2C(O)-$ ou $-CH_2CHOH-$) ou un groupe $-(CH_2)_3C(CH_3)_2-$, un groupe $-OCH_2CH_2C(CH_3)_2-$, un groupe - $C(CH_3)_2CH(CH_3)(CH_2C(CH_3)_2$- ou un groupe - $NHC(O)CH_2C(CH_3)_2-$, $R^4$ et $R^5$, lorsque $R^1$ à $R^3$ sont de l'hydrogène, formant ensemble un cycle du type cité ou $R^4$ représentant un reste alcoxy en $C_4$-$C_6$ ou alcoxyalkyle ramifié,

$R^6$ un atome d'hydrogène, le reste méthyle, éthyle ou cyclopropyle,

m et n, différents l'un de l'autre, 0 ou 1,

X un atome d'hydrogène, un groupe nitro, méthoxy ou nitrile, le reste acide sulfonique ou les restes - $CONR^7OR^7$, $CO_2R^7$, $-PO(OR^8)_2$, $S(O)_nR^8$ (avec n = 0, 1 ou 2), $-SO_2-NR^9R^{10}$ ou $-CONR^9R^{10}$ où $R^7$ représente hydrogène, un groupe alkyle en $C_1$-$C_3$ ou un groupe phényle, éventuellement substitué par un ou deux groupes amino, acylamino en $C_1$-$C_4$, alkyle ou alcoxy en C1-C4, et $R^8$ représente un groupe alkyle an $C_1$-$C_3$, $R^8$ et $R^9$, indépendamment l'un de l'autre, représentant hydrogène ou un groupe alkyle en $C_1$-$C_4$ ou formant ensemble un cycle pipéridine, pipérazine, morpholine ou thiomorpholine,

phénylhydrazones dans lesquelles, lorsque X est un atome d'hydrogène, un groupe méthoxy, nitrile ou nitro ou lorsque X = carboxyle et $R^1$ ou $R^2$ = hydroxyle, $R^4$ et $R^5$ forment, ensemble, un cycle du type cité plus haut ou $R^3$ et $R^5$ représentent, chacun, i-propyle ou iso- ou tert-butyle et si m = 0, $R^4$ ne représente pas le groupe hydroxyle, ainsi que leurs sels acceptables physiologiquement,

caractérisé par le fait que l'on met à réagir :

a) des composés carbonyles de formule IIa

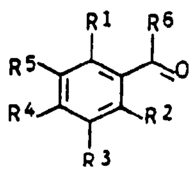

IIa,

dans laquelle $R^1$ à $R^6$ ont les significations données dans les revendications 1 à 6, avec des phénylhydrazines de formule IIIa

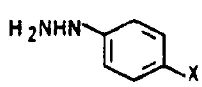

IIIa,

dans laquelle X a les significations données dans les revendications 1 à 6, ou

b) des composés carbonyles de formule IIb

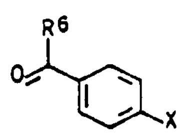

IIb,

dans laquelle $R^6$ et X ont les significations données plus haut, avec des phénylhydrazines de formule IIIb

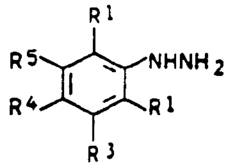

IIb,

dans laquelle $R^1$ à $R^5$ ont les significations données plus haut,

et l'on fait réagir le produit de réaction éventuellement avec un acide acceptable physiologiquement.

2. Procédé de préparation d'un médicament pour l'administration topique, caractérisé par le fait que l'on mélange des auxiliaires galéniques usuels avec 0,001 à 1 % en poids d'une phénylhydrazone de formule I, selon la revendication 1.

3. Procédé de préparation d'un médicament pour l'administration systémique, caractérisé par la fait que l'on mélange des auxiliaires galéniques usuels avec 0,1 à 250 mg d'une phénylhydrazone de formule I, selon la revendication 1.

4. Procédé de préparation d'une préparation cosmétique, caractérisé par le fait que l'on mélange des auxiliaires cosmétiques usuels avec 0,001 à 1 % en poids d'une phénylhydrazone de formule I, selon la revendication 1.

5. Procédé selon la revendication 2 pour la préparation d'un médicament pour le traitement de l'acné ou du psoriasis ou d'autres maladies dermatologiques accompagnées de la formation de callosités pathologiques, ainsi que d'eczémas, verrues et vitiligo.

6. Procédé selon la revendication 2 pour la préparation d'un médicament pour le traitement de lésions iatrogènes de la peau ou de lésions causées par la lumière UV.

7. Procédé selon la revendication 2 pour la préparation d'un médicament pour le traitement d'yeux secs et d'autres cornéopathies.

8. Procédé selon la revendication 2 pour la préparation d'un médicament pour le traitement de précancéroses et de tumeurs.

9. Procédé selon la revendication 2 pour la préparation d'un médicament pour le traitement des rhumatismes et de l'arthrite.

10. Préparation cosmétique contenant, outre les auxiliaires cosmétiques usuels, 0,001 à 1 % en poids d'une phénylhydrazone de formule I, selon la revendication 1.